Europäisches Patentamt

European Patent Office

Office européen des brevets

(11) Publication number: **0 347 190**
**A1**

# (12) EUROPEAN PATENT APPLICATION

(21) Application number: **89305995.6**

(51) Int. Cl.⁴: **A 61 M 5/30**

(22) Date of filing: **14.06.89**

(30) Priority: **14.06.88 US 206396**

(43) Date of publication of application:
**20.12.89 Bulletin 89/51**

(84) Designated Contracting States:
**BE DE ES FR GB IT**

(71) Applicant: **VCI CORPORATION**
**1912 Clearview Suite 102**
**Metairie Louisiana 70001 (US)**

(72) Inventor: **Finger, Richard F.**
**719 Atherton Drive Metairie**
**Louisiana 70001 (US)**

(74) Representative: **Valentine, Francis Anthony Brinsley et al**
**REDDIE & GROSE 16 Theobalds Road**
**London WC1X 8PL (GB)**

(54) **Needleless vacuum-compression injector.**

(57) A needleless injector has an injection nozzle (17) surrounded by a sleeve (14) to stabilize the nozzle (17) against tissue to be injected. Vacuum can be applied to the space within the sleeve (14) outside the nozzle (17). Medicament (16) is injected by means of a plunger (12) and a piston rod (25) of a piston and cylinder (20,23) to which a charge of compressed gas can be delivered. An interlock (24) allows delivery of such gas only if the vacuum in the sleeve (14) as sensed by a vacuum sensor (8) attains a predetermined value. The gas pressure may be adjusted to control depth of penetration of the injected medicament and the stroke length of the rod 25 may be adjusted (at 26) to control the volume of medicament injected.

FIG.1

EP 0 347 190 A1

**Description**

## NEEDLELESS VACUUM-COMPRESSION INJECTOR

This invention is in the field of needleless hypodermic and hypomucosal injectors using a vacuum to stabilize a nozzle of the device over the tissue to be penetrated.

The present invention provides a needleless vacuum-compression injector comprising:
medicament holding means for holding a medicament;
a vacuum generator for generating a vacuum and delivering said vacuum to a continuous vacuum system;
tissue stabilizing means in communication with said continuous vacuum system;
vacuum sensing means for sensing the vacuum in said system; a pressurized fluid source;
release means for said pressurized fluid;
actuation means connected to said pressurized fluid source and operative in response to the release of pressure by said release means for pressurizing and expelling said medicament from said medicament holding means; and,
locking means controlled by said vacuum sensing means for locking said actuation means against actuation until the vacuum in said system, and thereby at said tissue stabilizing means, reaches a predetermined value.

Needleless vacuum-compression injectors for hypodermic and hypomucosal applications are disclosed in U.S. Patent Nos. 4,403,609 and 4,421,508 to Cohen. These patents show regulation of vacuum to stabilize a nozzle of the device over the tissue to be penetrated. They further disclose compressed air pressure regulation to control penetration depth of the medicament into the tissue of the patient. The vacuum created in the region of the injector nozzle, however, is subject to various influences not always reliably under adequate control of the user. Moreover, the required vacuum level may not always be established at the instant of injector actuation. Hence, incomplete stabilization and sealing of the nozzle over the tissue to be injected may occur during injection.

The present invention provides salient improvements over existing injectors by interlocking injector actuation with the actual sensed presence of a desired vacuum level prior to initiation of medicament injection. The invention thusly ensures complete stabilization of the injector's nozzle over the tissue to be penetrated.

The foregoing and other objects, features and advantages of the invention will be apparent from the following more particular description of preferred embodiments of the invention, as illustrated in the accompanying drawings in which like reference characters refer to the same parts throughout different views. The drawings are schematic and not necessarily to scale, emphasis instead being placed upon illustrating principles of the invention.

FIG. 1 is a schematic representation of a longitudinal sectional view of an embodiment of a vacuum-interlocked, needleless, vacuum-compression injector according to principles of this invention;

FIG. 2 is a schematic representtion of a longitudinal sectional view of a hand-held portion of another embodiment of the injector of the present invention; and,

FIG. 3 is a schematic representation of a power and control-unit portion that is associated with the hand-held portion shown in FIG. 2.

A hitherto unsatisfied need has existed for injectors to be capable of injecting different amounts of medicament by prior preselection or adjustment of the mechanism, rather than having to be preloaded with specific amounts of medicament according to the particular momentary requirement or otherwise changing or selecting medicament content amount or volume of the medicament carrier.

A primary object of the invention, therefore, is to provide a needleless vacuum-compression injector which injects a preselected volume of medicament to a given depth only upon attainment of a desired vacuum level. ion; and, The device provides variable volume as well as pressure adjustment for the compressed gas used for driving the pneumatic injector piston to ensure adequate wide-range control over depth of medicament penetration. An adjustable piston stroke controls the amount of medicament injected.

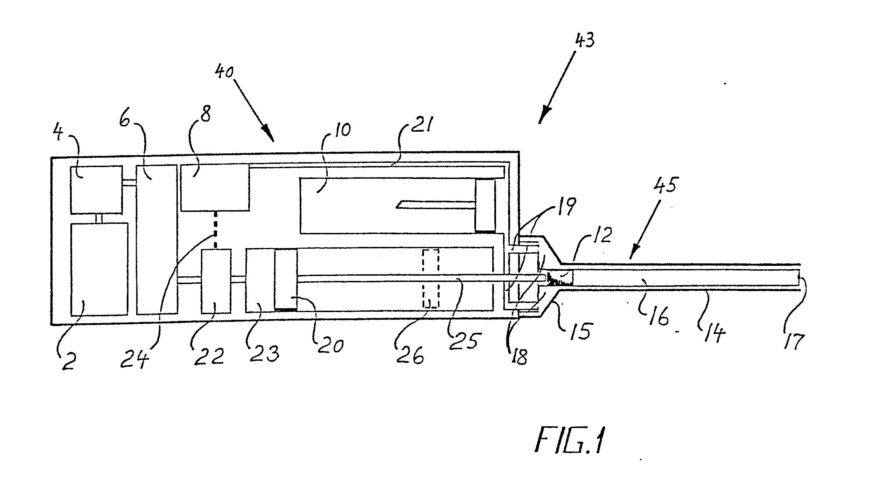

The schematic illustration shown in FIG. 1 represents a preferred embodiment of the injector device of the present invention in form of a self-contained, hand-held injector 40 comprising power and control unit 43 and medicament carrier and sleeve attachment 45. Attachment 45 comprises a medicament carrier 16 inserted within and surrounded by an outer sleeve 14. Sleeve 14 is provided with an enlarged base 15 that facilitates removal or uncoupling of attachment 45 from the power and control unit 43, for instance by means of appropriate mating threads. Medicament carrier 16 is filled with appropriate medicament prior to being attached within sleeve 14 to power and control unit 43. Medicament carrier 16 is of generally cylindrical shape having an axial bore terminating at its distal end in a small axial orifice nozzle 17 of appropriate dimension for needleless injection and having the bore near its proximal end sealed by a plunger plug 12.

The annular space between medicament carrier 16 and sleeve 14 permits vacuum-creating air flow from the region surrounding nozzle 17 at the distal end of medicament carrier 16 toward a vacuum generating device 10 via appropriate conduits 18 in base 15 and communicating conduits 19 that lead through the casing of power and control unit 43 to the vacuum generating device 10.

Vacuum generating device 10 may be, for instance, a spring-loaded piston/cylinder arrangement with a manual cocking mechanism (against spring) and an appropriate manual trigger or button for release actuation. The mechanism is of the general kind conventionally found in air guns and the like. In a preferred embodiment it is a double piston/cylinder arrangement powered by a compressed gas supply 2 such as a conventional compressed carbon dioxide gas cartridge which also powers piston 20 of cylinder 23.

Communication between the vacuum generating device 10 and conduits 18 and 19 are appropriately valved by valves not shown. The vacuum generating device 10 and conduits 18 and 19 may also be appropriately valved. The vacuum generating device 10 is adjustably variable by conventional means to provide different desired vacuum pressures. Conduits 19 further communicate via vacuum sensing conduit 21 with vacuum sensor 8 which utilizes a diaphragm or a piston to provide a mechanical output to an interlock coupling 24.

The injector power and control unit 43 further comprises a pneumatic cylinder 23 having a piston 20 which, when actuated, drives an attached piston rod 25 against plunger plug 12 and forces the plug 12 deeper into the bore of medicament carrier 16, thus pressurizing and expelling its medicament content through nozzle 17. A mechanically adjustable stop 26 (indicated by dashed lines) provides for piston stroke length adjustment and thus controls the amount of medicament displaced from the carrier 16 and injected into bodily tissues.

Pneumatic power for actuation of piston 20 of cylinder 23 is provided by a supply of compressed fluid, such as compressed gas 2, through a variable-pressure control 4 and via a variable volume chamber 6. The compressed gas 2 is released on demand to cylinder 23 by a release valve 22. Gas pressure within chamber 6 may also be sensed by an appropriate sensor. Thus compressed gas of a preselected volume and pressure is controlled by release valve 22 which is mechanically interlocked by means of the interlock coupling 24 (indicated by a dashed line) with the vacuum pressure sensed by vacuum sensor 8. The mechanism of interlock coupling 24 is suitably adjustable with respect to a desired minimum vacuum value to selectively provide an interlock-release or interlock-deactivation. For instance, the arrangement of vacuum sensor 8 and release valve 22, interlocked by mechanical coupling 24, is preferably provided by a vacuum-sensing piston/cylinder device that is adjustably spring loaded for adjustment of the desired vacuum level for triggering of a conventional miniature spool valve which is the release valve 22.

In preparation for use, the hand-held injector 40 is loaded with its medicament carrier 16 that is appropriately loaded with medicament. The injector is then adjusted to a desired vacuum interlock level by adjusting the coupling 24 and/or the valve 22 so that valve 22 only releases the gas from chamber 6 when the desired vacuum is sensed by sensor 8. In this respect, the actuation gas pressure is adjusted by means of the variable pressure control 4 and the actuation gas volume is controlled by the variable-volume chamber 6. Pressure control 4 may be a conventional miniature adjustable pressure regulator and variable-volume chamber 6 comprising an adjustable cylinder arrangement that is adjusted for instance by means of a screw-operated plunger. Similarly, the variable stroke length of piston 20 is adjusted by selective positioning of the stop 26.

In use, the vacuum generator 10 is actuated and the hand-held injector 40 is placed with nozzle 17 against the skin at the injection site. When outer sleeve 14 is sealed against the skin at its distal end, the vacuum created in the annular region between sleeve 14 and medicament carrier 16 is sensed by vacuum sensor 8. Provided that the desired preselected vacuum level is attained, the nozzle 17 is adequately stabilized over the tissue to be injected. Release valve 22 then opens and piston 20 in cylinder 23 is driven by the compressed gas toward the right. The piston rod 25 then forces plunger plug 12 into the medicament carrier 16 which is thusly pressurized and expels its medicament content through nozzle 17 into the tissue.

The piston rod 25 and plug 12 travel only for a distance that depends on the setting of stop 26. Consequently, commensurate amounts of medicament are expelled from medicament carrier 16 into the tissue. Hence, any dosage of medicament up to the full capacity of the carrier may be preselected, as for instance may be required by the dosage requirements corresponding to a particular patient's body weight.

It will be appreciated that the capability for providing an adjustable volume of compressed gas at an adjustable pressure to drive piston 20 and plunger plug 12 and therewith expel the medicament through nozzle 17 facilitates improved control over depth of penetration into bodily tissues. In this manner the relationship between instantaneous injection pressures and the amount of medicament to be injected during an injection stroke may be appropriately tailored.

In one embodiment of the invention the volume of chamber 6 is varied by means of a screw-operated plunger (not shown); and the variable pressure controller 4 is a conventional screw-operated diaphragm-type device. The two adjustments of pressure and volume, however, can be coupled to a single screw or slide mechanism without significant loss of accuracy so long as the diaphragm of the pressure regulator and the plunger of the volume control are appropriately sized.

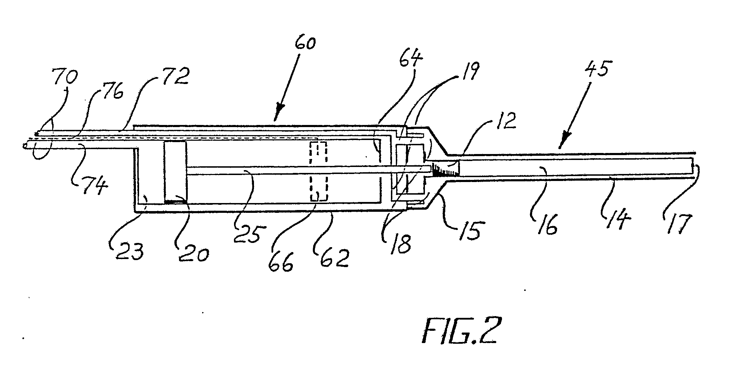

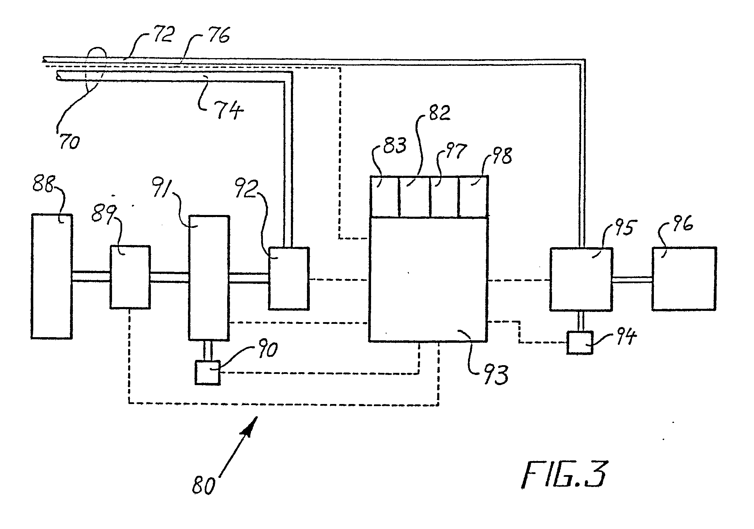

Another preferred embodiment shown schematically in FIGS. 2 and 3 represents the injector device of the present invention consisting of a hand unit 60 and a separate remote unit 80 which are interconnected by an umbilical cable 70.

As schematically illustrated in FIG. 2, the hand unit 60 comprises the medicament carrier 16 within the sleeve attachment 45 and the pneumatic cylinder 23 is encased within a cylinder housing 62. In this respect, the medicament carrier 16 is inserted within and surrounded by outer sleeve 14 which, as in the first embodiment, is provided with an enlarged base 15 which facilitates removal or uncoupling of attachment 45 from the cylinder housing 62, such as by mating threads.

The annular space between the medicament carrier 16 and the sleeve 14 permits vacuum-creating air flow from the region surrounding nozzle 17 into vacuum cable conduit 72 (in umbilical cable 70) via appropriate conduits 18 in base 15 and communicating conduits 19 that lead through cylinder housing 62.

The pneumatic cylinder 23 again comprises a piston 20 which, when actuated, drives the attached piston rod 25 against plunger plug 12 and forces it deeper into the bore of medicament carrier 16 to thusly pressurize and expel its medicament content through nozzle 17 and into the tissue. Pneumatic cylinder 23 further comprises an electromechanically adjustable stop 66 (indicated by dashed lines), located between piston 20 and cylinder end 64, that provides for piston-stroke-length adjustment and thusly controls the amount of medicament displaced from the carrier 16 and injected into bodily tissues. Position adjustment of stop 66 is controlled electromechanically by means of electrical signals delivered to a servo motor (not shown) through a stop-control line 76 (indicated by a dashed line) within the umbilical cable 70. Similarly, the pneumatic cylinder 23 is actuated and powered by pneumatic pressure fed to it through a pneumatic-cable conduit 74 in the umbilical cable 70.

FIG. 3 schematically illustrates the remote unit 80 which contains a power supply and control facilities for the FIG. 2 injector. As illustrated, the remote unit 80 is interconnected with the hand unit 60 via umbilical cable 70.

Remote unit 80 comprises a vacuum generator 96 connected to a vacuum control valve 95 which is controlled by an electronic control unit 93 comprising a commercial microprocessor with appropriate interfacing. In one embodiment of the invention, vacuum generator 96 utilizes compressed gas source 88 in the form of a compressed carbon dioxide cartridge or tank to produce the required vacuum by means of a conventional venturi ejector device. In another embodiment, vacuum generator 96 is a standard, commercially available, miniature vacuum pump.

In one preferred embodiment of the invention, vacuum control valve 95 comprises a plurality of miniature solenoid valves, each coupled with a miniature manually preadjustable needle valve acting as a bleed valve through appropriately calibrated different orifices, thus being capable of creating different vacuum levels. In this respect, commercially available solenoid valves from the Lee Company, model LFAA0503118H, and needle valves from the Clippard Instrument Labs, model MNV-1, are used. However, similar devices may be applied with equal success. Also an electrically actuatable and manually or electrically adjustable vacuum pressure regulator and valve may be utilized instead.

A conventional vacuum-sensing transducer 94 connects to and senses the vacuum pressure at the vacuum control valve 95 and provides the appropriate signal representative of that pressure to the control unit 93.

Transducer 94 can be a commerical vacuum-sensing transducer sold by Omega Engineering Co., model P241-05NG5V (0 to 20" Hg vacuum). Equivalent devices may also be used.

The desired vacuum level is adjustable by the operator via an "up-down" electronic vacuum selector 98 incorporated within the control unit 93. In this manner, selector 98 controls valve 95 which, in turn, controls the vacuum provided by generator 96; and, that vacuum, which is sensed by transducer 94 for delivery of a feedback signal to control unit 93, is in communication with the hand unit 60 via conduit 72 in umbilical cable 70.

Remote unit 80 further comprises a compressed gas source 88; an electronically adjustable pressure control device 89; an electronically adjustable, variable volume chamber 91; an electronic pressure sensor 90 for sensing pressure in chamber 91; and, a release valve 92. In this respect, the pressure control device 89 is a conventional motorized pressure regulator. It may be, however, as simple as a miniature solenoid valve. Either device is controlled by control unit 93 in dependence on pressure sensed in chamber 91 by pressure sensor 90. Pressure transducer model PX305-500GI (0 to 500 psi ) by Omega Engineering Co. has been used for the latter, but these conventional devices do not form part of the invention and, therefore, will not be further described.

Electronic control unit 93 receives the pressure signals from sensor 90 and provides the required control signals to pressure control device 89; to variable-volume chamber 91; and to release valve 92 such as a commercialy available miniature solenoid valve. In a manner corresponding to the first embodiment, the pneumatic pressure released by valve 92 is then fed to the hand unit 60 via conduit 74 in umbilical cable 70.

The desired injection pressure is adjustable by the operator via another "up-down" electronic pressure selector 97 located on the control unit 93. The desired volume of chamber 91 is also adjustable by the operator via yet another "up-down" selector 82 that is also located on the control unit 93. In this regard, as in the first embodiment, the volume selector 82 and the pressure selector 97 may be appropriately coupled within the electronic control unit 93 to provide a single adjustment selector for control of injection depth. In addition, as noted above, adjustment of stop 66 (FIG. 2) is preselected by the operator via dosage selector 83 located on the control unit 93. That is, appropriate control signals from control unit 93 are provided via the stop control connection 76 within umbilical cable 70 to the servo-motor for the adjustable stop 66 in hand unit 60.

In preparation for use, the injector hand unit 60 is loaded with its medicament carrier 16 which is filled with appropriate medicament. Vacuum selector 98 on the remote unit 80 is then adjusted to select the desired vacuum interlock level to be sensed by vacuum-sensing transducer 94. Appropriate injection penetration pressure selector 97 and volume selector 82, and the appropriate medicament dosage is chosen by adjustment of dosage selector 83.

In this respect, selectors 83, 82, 97, and 98 are appropriate manually operatable "up-down" switches that provide commands to electronic control unit 93, as described. For instance, selector 83, via unit 93, commands a servo-motor arrangement that adjusts adjustable stop 66 (FIG. 2) by means of a motorized screw mechanism.

The hand unit 60 is then placed with nozzle 17 against the skin at the injection site. When the distal end of outer sleeve 14 is sealed against the skin, the vacuum created in the annular region between sleeve 14 and medicament carrier 16 is communicated via the umbilical cable 70 to the remote unit 80 and is sensed by the vacuum sensing transducer 94 as described above. Provided that the preselected vacuum interlock level is attained (indicating adequate stabilization of nozzle 17 over the tissue to be injected) the release valve 92 opens and pneumatic pressure feeds through conduit 74 in umbilical cable 70 to cylinder 23 in the hand unit 60. Piston 20 in cylinder 23 is thereby driven by compressed gas toward the right in FIG. 2. The piston rod 25 then forces the plunger plug 12 into the medicament carrier 16 to thus presurize and expel the medicament content through nozzle 17 and into the desired tissue to the desired depth. In this respect, the piston rod 25 and plunger plug 12 travel for a distance limited by the preselected setting of stop 66 so that only the preselected dosage of medicament is expelled from medicament carrier 16.

While the invention has been particularly shown and described with reference to preferred embodiments thereof, it will be understood by those skilled in the art that various changes and modifications in form and details may be made therein without departing from the spirit and scope of the invention. The controls on the remote unit 80, for example, have been described in terms of a preferred embodiment employing otherwise conventional electronic controls. Mechanical controls, however, such as used in the first embodiment can also be employed if the conveniences of electronic controls are for some reason not desired.

## Claims

1. A needleless vacuum-compression injector comprising:
medicament holding means for holding a medicament;
a vacuum generator for generating a vacuum and delivering said vacuum to a continuous vacuum system;
tissue stabilizing means in communication with said continuous vacuum system;
vacuum sensing means for sensing the vacuum in said system;
a pressurized fluid source;
release means for said pressurized fluid;
actuation means connected to said pressurized fluid source and operative in response to the release of pressure by said release means for pressurizing and expelling said medicament from said medicament holding means; and,
locking means controlled by said vacuum sensing means for locking said actuation means against actuation until the vacuum in said system, and thereby at said tissue stabilizing means, reaches a predetermined value.

2. Apparatus of claim 1 including means for sensing the pressure of said pressurized fluid and means for regulating the pressure of said pressurized fluid.

3. Apparatus of claim 1 including means for selectively varying the vacuum pressure at which said locking means permits actuation of said actuation means.

4. Apparatus of claim 1 including means for varying the volume of said pressurized fluid that is available to drive said actuation means upon actuation thereof.

5. Apparatus of claim 1 wherein said actuation means includes a pneumatic cylinder and piston; and,
means for adjusting said piston's stroke length, whereby said piston's stroke length controls the amount of medicament that is expelled by said injector.

6. A needleless vacuum-compression injector for dispensing a medicament into a tissue body, said needleless vacuum-compression injector comprising:
a medicament holding means for holding a quantity of medicament, said medicament holding means having a medicament expelling port which is positioned proximate said tissue body;
a tissue stabilizing means which surrounds said medicament expelling port of said medicament holding means, said tissue stabilizng means being positioned proximate said tissue body;
means for generating a vacuum, said vacuum being delivered to said tissue stabilizng means

so as to create a seal between said tissue body and said tissue stabilizing means whereby said tissue body is prevented from moving with respect to said medicament expelling port; means for sensing the amount of vacuum generated in said tissue stabilizing means; a supply of pressurized fluid; means for releasing said fluid in response to the attainment of a desired amount of vacuum in said tissue stabilizing means; actuation means operative in response to the release of said pressurized fluid by said releasing means for pressurizing and expelling said medicament from said medicament expelling port; and, locking means controlled by said vacuum sensing means for locking said actuation means against actuation until the amount of vacuum in said tissue stabilizing means reaches a predetermined value.

7. The apparatus of claim 8 wherein the amount of fluid supplied to said actuation means is controlled by a variable volume chamber.

8. The apparatus of claim 8 wherein said needleless vacuum-compression injector comprises a hand unit and a remote unit, said hand unit containing said medicament holding means, said tissue stabilizing means and said actuation means, said hand unit being interfaced with said vacuum generating means and said supply of pressurized fluid contained in said remote unit by means of a multi-conduit umbilical cable.

9. A needleless vacuum-compression injector for dispensing a medicament into a tissue body, said needleless vacuum-compression injector comprising:
medicament holding means for holding a quantity of a medicament, said medicament holding means having an orifice nozzle at its distal end, from which said medicament is expelled into said tissue body;
tissue stabilizng means surounding said orifice nozzle;
vacuum generating means, said vacuum generating means delivering said vacuum to said tissue stabilizing means via a vacuum conduit so that said tissue stabilizing means can accurately position said orifice nozzle on said tissue body;
means for adjusting the amount of vacuum produced by said vacuum generating means;
a vacuum sensor for determining the amount of vacuum produced by said vacuum generating means;
a source of pressurized fluid;
means for adjusting the pressure of said pressurized fluid;
fluid releasing means for releasing said fluid in response to the attainment of a desired amount of vacuum;
a pneumatic cylinder which receives said pressurized fluid so as to expel said medicament into said tissue body;
means for adjusting the volume of fluid delivered to said pneumatic cylinder;
means for limiting the amount of medicament which is expelled from said orifice nozzle so as to control the amount of medicament injected into said tissue body; and,
an interlock coupler for ensuring that said pressurized fluid is not supplied to said pneumatic cylinder prior to the attainment of a preselected amount of vacuum, the attainment of said preselected amount of vacuum indicating that said orifice nozzle has been properly positioned with respect to said tissue body.

10. A method of injecting a selected amount of medicament into a tissue body using a needleless vacuum-compression injector, comprising the steps of:
filling an orifice nozzle of a medicament carrier with an appropriate amount of medicament;
positioning the orifice nozzle on a desired section of a tissue body;
immobilizing the movement of the tissue body by applying a vacuum to the tissue body at the point where the orifice nozzle contacts the tissue body;
selecting the desired amount of vacuum to be applied to the tissue body;
sensing the amount of vacuum applied to the tissue body;
controlling the amount of medicament that is expelled from the orifice nozzle; and,
expelling the medicament from the medicament carrier into the tissue body after the tissue body has been immobilized.

FIG.1

FIG.2

EP 0 347 190 A1

FIG.3

EP 0 347 190 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | US-A-4 403 609 (COHEN)<br>* Figure 1 * | 1 | A 61 M 5/30 |
| A,D | US-A-4 421 508 (COHEN)<br>* Figure 1 * | 1 | |

TECHNICAL FIELDS SEARCHED (Int. Cl.4)

A 61 M

The present search report has been drawn up for all claims

| Place of search | Date of completion of the search | Examiner |
|---|---|---|
| THE HAGUE | 15-09-1989 | SEDY, R. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)